# EUROPEAN PATENT APPLICATION

(11) **EP 1 216 625 A1**
(43) Date of publication of application: **26.06.2002**
(21) Application number: 00870309.2
(22) Date of filing: 21.12.2000
(51) Int. Cl.: A23L 1/308, A23L 1/30, A61K 31/715

(54) **The use of a non-absorbable, non-digestible lipid for the treatment of hyperbilirubinemia**

(71) Applicant: THE PROCTER & GAMBLE COMPANY, Cincinnati, Ohio 45202 (US)
(72) Inventor: Kotal, Petr, 142 00 Prague 4 (CZ); Vitek, Libor, 128 08 Prague 2 (CZ)
(74) Representative: Canonici, Jean-Jacques

(57) **Abstract**

Described is the use of a non-absorbable, non-digestible lipid, for the manufacture of a pharmaceutical or dietary composition for the treatment of hyperbilirubinemia, specifically unconjugated hyperbilirubinemia such as neonatal jaundice, Crigler-Najjar syndrom and Gilbert syndrom.

## Description

The present invention relates to the use of a non-absorbable, nondigestible lipid for the manufacture of a pharmaceutical or dietary composition for the treatment of hyperbilirubinemia, specifically unconjugated hyperbilirubinemia in neonates, Crigler-Najjar syndrom and Gilbert syndrom.

Bilirubin is an end product of heam catabolism, which is disposed via bile into the intestinal lumen in man in a relatively constant amount of about 500 µmol a day.

Unconjugated bilirubin gets partially recirculated in the enterohepatic cycle and enhances systemic bilirubin levels. In a pathological condition, unconjugated bilirubin accumulates in the body, including the intestinal lumen, and may induce toxic effects in different tissues, especially in the neonate, in whom this can lead to bilirubin encephalopathy. Actually, neonates are not able to detoxify bilirubin by its conversion to urobilinogen and thus accumulate unconjugated bilirubin in the intestinal lumen. Today, about 10 % of born neonates world-wide suffer from an excessive level of serum bilirubin causing pathological neonatal jaundice. So far, mostly low-efficient phototherapy, which requires hospitalisation, is applied and in severe cases transfusion is offered. In addition, the inefficient biliary excretion of bilirubin (i.e. in Crigler-Najjar patients and neonates) increases this pathological situation called hyperbilirubinemia.

Thus, there is an urgent need to provide for a simpler and faster therapy of this excessive level of serum bilirubin.

It is already known from JP-62-141576 that bilirubin contained in serum can be adsorbed from a porous copolymer consisting of divinyl benzene and glycidyl methacrylate.

On the other hand it has been described that Olestra, i.e. a nonabsorb- able, non-digestible polyol fatty acid polyester can be used to speed up the removal of dioxins and PCBs from the body (New Scientist (1999), 26, Lancet (1999), 1266-1267, Chemosphere (1999), 1513-1521 and US-A-4,241,054).

It has now been surprisingly found that it is possible to eliminate excess bilirubin from the serum by partition of bilirubin into a lipid in the digestive tract and the subsequent elimination out of the body together with stools. This partition of bilirubin into said lipid interferes with entero-hepatic and entero-enteric circulation of bilirubin and provides for a substantial reduction of the serum bilirubin level and thus the possibility of the treatment of hyperbilirubinemia.

The present invention therefore relates to the use of a non-absorbable, non-digestible lipid, for the manufacture of a pharmaceutical composition for the treatment of hyperbilirubinemia, specifically unconjugated hyperbilirubinemia, such as neonatal jaundice, Crigler-Najjar syndrom and Gilbert syndrom.

According to a preferred embodiment of the present invention, the non-absorbable, non-digestible lipid is selected from the group consisting of polyol fatty acid polyesters, esters of tricarballylic acids, diesters of dicarboxylic acids, triglycerides of alpha-branched chain carboxylic acids, ethers and ether esters containing the neopentyl moiety, fatty polyethers of polyglycerol, alkyl glycoside fatty acid polyesters, polyesters of two ether linked hydroxypolycarboxylic acids, polydimethyl siloxanes, esterified linked alkoxylated glycerols, and mixtures thereof.

Preferably, the non-absorbable, non-digestible polyol fatty acid polyester is a polyol fatty acid polyester having at least 4 fatty acid ester groups, wherein the polyol is selected from the group consisting of sugars and sugar alcohols containing from 4 to 8 hydroxyl groups and wherein each fatty acid group has from about 8 to about 22 carbon atoms.

Advantageously, the polyol fatty acid polyester contains no more than 2 free hydroxyl groups, while the fatty acid ester groups preferably contain from about 14 to 18 carbon atoms. According to a further preferred embodiment the polyol is a member selected from the group consisting of erythritol, xylitol, sorbitol, glucose and sucrose. Most preferably the polyol is sucrose.

Desirably the sucrose fatty acid polyester is a member selected from the group consisting of C₁₄, C₁₆, and C₁₈ hexa-, hepta-, and octaesters, and mixtures thereof. More preferred are polyol fatty acid polyesters are selected from the group consisting of polyol fatty acid polyesters that comprise sugar polyesters, sugar alcohol polyesters, and mixtures thereof. Of these those are especially preferred wherein the sugars and sugar alcohols of the non-absorbable, non-digestible sugar polyesters and sugar alcohol polyesters are selected from the group consisting of erythritol, xylitol, sorbitol, glucose, and sucrose.

Further preferred examples of non-absorbable, non-digestible polyol fatty acid polyestes is selected from the group consisting of sucrose tetraoleate, sucrose pentaoleate, sucrose hexaoleate, sucrose heptaoleate, sucrose octaoleate, sucrose hepta- and octaesters of unsaturated soybean oil fatty acids, canola oil fatty acids, cottonseed oil fatty acids, corn oil fatty acids, peanut oil fatty acids, palm kernel oil fatty acids, or coconut oil fatty acids, glucose tetraoleate, the glucose tetraesters of coconut oil or unsaturated soybean oil fatty acids, the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, and mixtures thereof.

Most preferably the sucrose fatty acid polyester is a member selected from the group consisting of the hexaoleate, heptaoleate and octaoleate of sucrose, and mixtures thereof.

In dependency from the chain length of the fatty acid groups, said polyol fatty acid polyesters can be liquid or solid.

Nondigestible polyol polyesters that are liquid at body temperature are those which have minimal or no solids at body temperatures (i.e., 37°C). These liquid polyol polyesters typically contain ester groups having a high proportion of C₁₂ or lower saturated fatty acid radicals or else a high proportion of C₁₈ or higher unsaturated fatty acid radicals. In the case of those liquid polyol polyesters having high proportions of unsaturated C₁₈ or higher fatty acid radicals, at least about half of the fatty acids incorporated into the polyester molecule are typically unsaturated. Preferred unsaturated fatty acids in such liquid polyol polyesters are oleic acid, linoleic acid, and mixtures thereof.

Specifically preferred according to the present invention is the use of a mixture of a liquid polyol fatty acid polyester as defined above and of a solid polyol fatty acid polyester having a complete melting point above about 37°C, wherein a) the polyol has at least 4 hydroxyl groups, b) the ester groups of the solid polyol fatty acid polyester comprise (i) C₁₂ or higher unsaturated fatty acid radicals or a mixture of said unsaturated radicals and C₂ to C₁₂ saturated fatty acid radicals, and (ii) C₂₀ or higher saturated fatty acid radicals, the molar ratio of (i):(ii) is from about 1:15 to about 2:1, such as approximately 1.5:6.5, and c) at least 4 of the hydroxyl groups of the polyol are esterified, said mixture being semi-solid at body temperature to control oil loss, i.e.anal leakage and thus having a complete melting point above about 37°C. Most preferably said mixture comprises 50 % to 98 % per weight, preferably 90 % to 95 % per weight of said liquid polyol fatty acid polyester, and 50 % to 2 % by weight, preferably 10 % to 5 % per weight of said solid polyol fatty acid polyester.

These non-absorbable, non-digestible polyol fatty acid polyesters are known and described in the prior art, such as US-patents 3,600,186 and 5,306,514, to which herewith specific reference is made. Furthermore, the preferred non-absorbable, non-digestible polyol fatty acid polyester used in the present invention is available on the market under the brand name Olestra® by the applicant. This product is an admixture of a liquid polyol polyester component and a polyol polyester component to provide a semi-solid consistency with a complete melting point above about 37°C. Preferably this consistency is obtained by blending approximately 6 wt.-% of said solid polyol polyester component with approximately 94 wt.-% of said liquid polyol polyester component.

The pharmaceutical or dietary compositions provided according to the use of this invention comprise well-defined polyol fatty acid esters. Suitable nondigestible edible lipids for use herein include polyol fatty acid polyesters (see Jandacek; U.S. Pat. No. 4,005,195), esters of tricarballylic acids (see Hamm; U.S. Pat. No. 4,508,746), diesters of dicarboxylic acids such as derivatives of malonic and succinic acid (see Fulcher, U.S. Pat. No. 4,582,927; triglycerides of alpha-branched chain carboxylic acids (see Whyte; U.S. Pat. No. 3,579,548), ethers and ether esters containing the neopentyl moiety (see Minich; U.S. Pat. No. 2,962,419), fatty polyethers of polyglycerol (See Hunter et al; U.S. Pat. No. 3,932,532), alkyl glycoside fatty acid polyesters (see Meyer et al; U.S. Pat. No. 4,840,815), polyesters of two ether linked hydroxypolycarboxylic acids (e.g., citric or isocitric acid) (see Huhn et al; U.S. Pat. No. 4,888,195), and esters of epoxide-extended polyols (see White et al; U.S. Pat. No. 4,861,613), as well as polydimethyl siloxanes (e.g., Fluid Silicones available from Dow Corning). All of the foregoing patents relating to the nondigestible lipid component are incorporated herein by reference.

Preferred nondigestible lipids are the polyol fatty acid polyesters that comprise sugar polyesters, sugar alcohol polyesters, and mixtures thereof. The preferred sugars and sugar alcohols for preparing these polyol polyesters include erythritol, xylitol, sorbitol, glucose, and sucrose, with sucrose being especially preferred. The sugar or sugar alcohol starting materials for these polyol polyesters are preferably esterified with fatty acids containing from 8 to 22 carbon atoms, and most preferably from 8 to 18 carbon atoms. When sucrose is used to prepare the polyol fatty acid polyesters, the resulting sucrose polyester has on average at least 4, preferably at least 5, fatty acid ester linkages per molecule.

Fatty acids per se or naturally occurring fats and oils can serve as the source for the fatty acid component in the polyol fatty acid ester. For example, rapeseed oil provides a good source for C₂₂ fatty acid. C₁₆-C₁₈ fatty acid can be provided by tallow, soybean oil, canola oil (i.e. fatty acids derived from low erucic acid rapeseed oil), or cottonseed oil. Shorter chain fatty acids can be provided by coconut, palm kernel, or babassu oils. Corn oil, lard, olive oil, palm oil, peanut oil, safflower seed oil, sesame seed oil, and sunflower seed oil, are examples of other natural oils which can serve as the source of the fatty acid component. Among the fatty acids, those that are preferred have from about 14 to about 18 carbon atoms, and are most preferably selected from the group consisting of myristic, palmitic, stearic, oleic, and linoleic fatty acids. Thus, natural fats and oils which have a high content of these fatty acids represent preferred sources for the fatty acid components, e.g., soybean oil, olive oil, cottonseed oil, corn oil, tallow and lard.

Other suitable polyol fatty acid polyesters are esterified linked alkoxylated glycerols, including those comprising polyether glycol linking segments, as described in U.S. Patent No. 5,374,446, incorporated herein by reference, and those comprising polycarboxylate linking segments, as described in U. S. Patent Nos. 5,427,815 and 5,516,544, incorporated herein by reference; more preferred are those described in U. S. Patent No. 5,516,544.

Other suitable esterified epoxide-extended polyols include esterified alkoxylated polysaccharides. Preferred esterified alkoxylated polysaccharides are esterified alkoxylated polysaccharides containing anhydromonosaccharide units, more preferred are esterified propoxylated polysaccharides containing anhydromonosaccharide units, as described in U. S. Patent No. 5,273,772, incorporated herein by reference.

Additional suitable polyol fatty acid polyesters are esterified epoxide-extended polyols of the general formula P(OH)A+C (EPO)N (FE)B wherein P(OH) is a polyol, A is from 2 to about 8 primary hydroxyls, C is from about 0 to about 8 total secondary and tertiary hydroxyls, A + C is from about 3 to about 8, EPO is a C₃- C₆ epoxide, N is a minimum epoxylation index average number, FE is a fatty acid acyl moiety and B is an average number is the range of greater than 2 and no greater than A + C, as described in U. S. Patent No. 4,861,613 and EP 0324010 A1, incorporated herein by reference. The minimum epoxylation index average number has a value generally equal to or greater than A and is a number sufficient so that greater than 95% of the primary hydroxyls of the polyol are converted to secondary or tertiary hydroxyls. Preferably the fatty acid acyl moiety has a C₇ -C₂₃ alkyl chain.

Preferred esterified epoxide-extended polyols include esterified propoxylated glycerols prepared by reacting a propoxylated glycerol having from 2 to 100 oxypropylene units per glycerol with C₁₀ -C₂₄ fatty acids or with C₁₀ -C₂₄ fatty acid esters, as described in U. S. Patent Nos. 4,983,329 and 5,175,323, respectively. Also preferred are esterified propoxylated glycerols prepared by reacting an epoxide and a triglyceride with an aliphatic polyalcohol, as described in U. S. Patent No. 5,304,665, or with an alkali metal or alkaline earth salt of an aliphatic alcohol, as described in U. S. Patent No. 5,399,728. More preferred are acylated propylene oxide-extended glycerols having a propoxylation index of above about 2, preferably in the range of from about 2 to about 8, more preferably about 5 or above, wherein the acyl groups are C₈ -C₂₄, preferably C₁₄- C₁₈, compounds, as described in U. S. Patent Nos. 5,603,978 and 5,641,534. Particularly preferred are fatty acid-esterified propoxylated glycerols which exhibit a sharp metal before about 33°C and have a dilatomeric solid fat index at 33°C of less than about 30, as described in WO 97/2260, or which have a dilatomeric solid fat index of at least about 50 at 21°C and at least about 10 at 37°C, as described in U. S. Patent Nos. 5,589,217 and 5,597,605.

The polyol fatty acid polyesters suitable for use in the compositions herein can be prepared by a variety of methods known to those skilled in the art. These methods include: transesterification of the polyol (i.e. sugar or sugar alcohol) with methyl, ethyl or glycerol esters containing the desired acid radicals using a variety of catalysts; acylation of the polyol with an acid chloride; acylation of the polyol with an acid anhydride; and acylation of the polyol with the desired acid, per se. (See, for example, U.S. Pat. Nos. 2,831,854, 3,600,186, 3,963,699, 4,517,360 and 4,518,772, all of which are incorporated by reference. These patents all disclose suitable methods for preparing polyol polyesters.)

Specific, but nonlimiting, examples of the preparation of polyol fatty acid polyesters suitable for use as non-absorbable, non-digestible lipids in the practice of the present invention are as follows:

Erythritol tetraoleate: Erythritol and a five-fold molar excess of methyl oleate are heated at 180°C. under vacuum, with agitation, in the presence of sodium methoxide catalyst over two reaction periods of several hours each. The reaction product (predominately erythritol tetraoleate) is refined in petroleum ether and crystallized three times from several volumes of acetone at 1°C.

Xylitol pentaoleate: Xylitol and a five-fold molar excess of methyl oleate in dimethylacetamide (DMAC) solution are heated at 180°C for five hours in the presence of sodium methoxide catalyst, under vacuum. During this time the DMAC is removed by distillation. The product (predominately xylitol pentaoleate) is refined in petroleum ether solution and, after being freed of petroleum ether, is separated as a liquid layer four times from acetone at about 1°C and twice from alcohol at about 10°C.

Sorbitol hexaoleate is prepared by essentially the same procedure used to prepare xylitol pentaoleate except that sorbitol is substituted for xylitol.

Sucrose octaoleate is prepared by substantially the same procedure as that used to prepare erythritol tetraoleate except that sucrose is substituted for erythritol. Sucrose octaesters of soybean oil fatty acids: Soybean oil is partially hydrogenated to an iodine value of 107 and then converted to the respective methyl esters. These methyl esters are then reacted with sucrose in the presence of a potassium carbonate catalyst and the potassium soap of the soybean oil fatty acids.

Sucrose octaesters of canola oil fatty acids: Canola oil is partially hydrogenated to an iodine value of 90 and then converted to the respective methyl esters. These methyl esters are then reacted with sucrose at about 135°C in the presence of a potassium carbonate catalyst and the potassium soap of the canola oil fatty acids. See Example 1 of U.S. Pat. No. 4,517,360 t. Sucrose octaesters of soybean hardstock/soybean oil fatty acids: See Examples 1 and 2 of European patent application 236,288 .

Sucrose octaesters of predominantly myristic acid: Myristic acid (at least 99% pure) is converted to the respective methyl esters. These methyl esters are then reacted with sucrose at about 135°C in the presence of a potassium carbonate catalyst and the potassium soap of myristic acid. See Example 2 (reaction conditions) and 1 (wash conditions) of U.S. Pat. No. 4,517,360.

Sucrose octaesters of palm kernel oil fatty acids: Palm kernel oil (hydrogenated to an iodine value of about 4) is converted to the respective methyl esters. These methyl esters are the respective methyl esters. These methyl esters are then reacted with sucrose at about 135°C in the presence of a potassium carbonate catalyst and the potassium soap of the palm kernel oil fatty acids. See Example 1 of U.S. Pat. No. 4,517,360.

The most preferred nondigestible lipid is the Olean® brand of olestra, which is sold by the Procter & Gamble Company of Cincinnati , Ohio U.S.A.

The polyol starting material preferably must have at least four esterifiable hydroxyl groups. Examples of suitable polyols are sugars, including monosaccharides and disaccharides, and sugar alcohols. Examples of monosaccharides containing four hydroxyl groups are xylose, and arabinose and the sugar alcohol derived from xylose, i.e., xylitol. The monosaccharide erythrose starting material is not suitable for the practice of this invention since it only contains three hydroxyl groups; but, the sugar alcohol derived from erythrose, i.e., erythritol, contains four hydroxyl groups and accordingly can be used. Suitable five hydroxyl group -containing monosaccharides are galactose, fructose, and sorbose. Six hydroxyl groups containing sugar alcohols derived from sucrose, glucose, and sorbose, e.g., sorbitol, are also suitable. Examples of disaccharide polyols which can be used include maltose, lactose, and sucrose, all of which contain eight hydroxyl groups.

The polyol starting material having at least four hydroxyl groups must be esterified with a fatty acid having from about eight to about 22 carbon a-toms. Examples of such fatty acids include caprylic, capric, lauric, myristic, myristoleic, palmitic, palmitoleic, stearic, oleic, ricinoleic, linoleic, linolenic, oleostearic, arachidic, arachidonic, behenic, and erucic acid. The fatty acids can be derived from naturally occurring or synthetic fatty aicds; they can be saturated or unsaturated, including positional and geometrical isomers, depending on the desired physical properties, for example liquid or solid, of the polyol fatty acid ester compound.

The polyol fatty acid esters useful in this invention preferably contain at least four fatty acid ester groups. Polyol fatty acid ester compounds that contain three or less fatty acid ester groups tend to be digested in the intestinal tract much in the manner as ordinary triglyceride fats and thus are not useful for trapping bilirubin within intestinal lumen, whereas the polyol fatty acid ester compounds that contain four or more fatty acid ester groups are substantially non-absorbable and non-digestible by the human body. It is not necessary that all of the hydroxyl groups of the polyol be esterified with fatty acid but it is preferable that the compound contain no more than two unesterified hydroxyl groups. Most preferably, all of the hydroxyl groups of the polyol are esterified with fatty acid, i.e., the compound is substantially completely esterified. The fatty acid ester groups can be the same or mixed on the same polyol molecule.

Thus, to illustrate the above points, sucrose triester of fatty acid would not be suitable for use herein because it does not contain the required four fatty acid ester groups. Sucrose tetra fatty acid ester would be suitable but is not preferred because it has more than two unesterified hydroxyl groups. Sucrose hexa fatty acid ester would be preferred because it has no more than two unesterified hydroxyl groups. An example of a highly preferred compound in which all of the hydroxyl groups are esterified with fatty acid is sucrose octa fatty acid ester. In any given polyol fatty acid ester compound, the fatty acid ester groups can be selected in view of the desired physical properties of the compound. The polyol polyester compounds which contain unsaturated fatty acid ester groups and/or a preponderance of short chain, (e.g., C₁₂ or shorter) fatty acid ester groups are generally liquid at room temperature. The polyol polyesters esterified with predominantly long (e.g., C₁₄ or longer) chain and/or saturated fatty acids, e.g., stearic (C18:0), are generally solids at room temperatures.

The following are examples of suitable polyol fatty acid esters containing at least four fatty acid ester groups, suitable for use in the present invention. Glucose tetraoleate, glucose tetrastearate, glucose tetraester of soybean oil fatty acid, mannose tetraester of tallow fatty acids, galactose tetraester of olive oil fatty acid, arabinose tetraester of cottonseed oil fatty acid, xylose tetralinoleate, galactose pentastearate, sorbitol tetraoleate, sorbitol hexaester of olive oil fatty acid, xylitol pentapalmitate, xylitol tetraester of substantially completely hydrogenated cottonseed oil fatty acid, sucrose tetrastearate, sucrose pentastearate, sucrose hexaoleate, sucrose octaoleate, sucrose octaester of substantially completely hydrogenated soybean oil fatty acid, sucrose octaester of peanut oil fatty acid. As noted before, highly preferred polyol fatty acid esters are those wherein the fatty acids contain from about 14 to about 18 carbon atoms and are thus derived from such natural materials as soybean oil and olive oil. Examples of such compounds are erythritol tetraester of olive oil fatty acid, erythritol tetraoleate, xylitol pentaoleate, sorbitol hexaoleate, sucrose octaoleate, and sucrose octaester of soybean oil fatty acid.

Erythritol tetraoleate -- Erythritol and a 5-fold excess of ethyl oleate was heated under vacuum during mechanical agitation, in the presence of sodium methoxide catalyst over 2 several hour periods at about 180°C. The reaction product (erythritol tetraoleate) was refined in petroleum ether and crystallized three times from several volumes of acetone at 1°C.

Sucrose octaoleate was also prepared by essentially the same procedure as that used to prepare xylitol pentaoleate except that sucrose was substituted for xylitol.

According to a further preferred embodiment of the present invention the pharmaceutical or dietary composition used further comprises sufficient fat-soluble vitamines selected from the group consisting of vitamine A, vitamine D, vitamine E and vitamine K, or mixtures thereof, to prevent abnormally low levels of any of said fat-soluble vitamines in the patients ingesting said composition.

Specifically in view of using the polyol fatty acid polyester in adults, it is preferred to incorporate in the pharmaceutical or dietary composition actually used a sufficient amount of an anti-anal leakage agent to prevent leakage of said polyol fatty acid polyester through the anal sphincter. This anti-anal leakage agent preferably is a member selected from the group consisting of: edible C₁₂ and higher saturated fatty acids, and their edible salts; edible, digestible sources of C₁₂ and higher saturated fatty acids; edible, non-absorbable, non-digestible solid polyol fatty acid polyesters having at least 4 fatty acid ester groups, wherein the polyol is selected from the group consisting of sugars and sugar alcohols containing from 4 to 8 hydroxyl groups and wherein each fatty acid group has from about 8 to about 22 carbon atoms; and edible, non-digestible esters of alpha-branched chain C₁₀-C₁₈ fatty acids.

More preferably, the anti-anal leakage agent is selected from the group consisting of edible C₁₂-C₂₄ saturated fatty acids and edible salts of C₁₂-C₂₄ saturated fatty acids and substantially saturated triglyceride esters of C₁₂-C₂₄ fatty acids, or mixtures thereof, preferably edible, position-specific trigylceride selected from the group consisting of: the 1-stearoyl, 1-palmitoyl, 1-arachidoyl and 1-behenoyl 2,3-dioleins; the 2-oleoyl 1,3-distearins, 1,3-dipalmitims, 1,3-diarachidins and 1,3-dibehenins; 1-oleoyl distearin; 1-palmitoyl distearin; 1-arachidoyl distearin; and 1-behenoyl distearin; and mixtures thereof. Most preferably selected from the group consisting of hydrogenated palm oil and natural or synthetic cocoa butter.

The pharmaceutical composition provided according to the use of the present invention preferably is in liquid form or in solid form having a complete melting point of above about 37°C and can be either in the form of a medicament or a dietary composition. Such medicament preferably is in the form of orally administrable drops, liquids or capsules, preferred are capsules made from gelatin, comprising said polyol fatty acid polyester or polyol fatty acid polyester mixture, said vitamines, said anti-anal leakage agent, and usual pharmaceutically inert, non-toxic excipients and additives; or in the form of a dietary preparation comprising said polyol fatty acid polyester or polyol fatty acid polyester mixture, and, if desired said vitamins, said anti-anal leakage agent, and usual pharmaceutically inert, non-toxic excipients and additives. Most preferably, said dietary preparation according to the use of the present invention is in the form of milk, artificial mother milk and/or infant formula substitute.

The required dosage of the polyol fatty acid esters will vary with the severity of the condition and the duration of the treatment. Dosages can range from about 0.1 mg/kg to about 500 mg/kg (unless otherwise specified, the unit designated "mg/kg" as used herein refers to mg of polyol fatty acid polyester per kilogram of body weight), preferably from about 5 mg/kg to about 125 mg/kg with up to six dosages, preferably up to four dosages daily.

As used herein, the term "pharmaceutical inert, non-toxic excipients and additives" denotes solid or liquid filler, diluent or encapsulating substan-ces. Some examples of the substances which can serve as pharmaceutical excipients or carriers are sugars such as lactose, glucose and sucrose; starches such as corn starch and potato starch; cellulose and its derivatives, such as sodium carboxymethylcellulose, ethylcellulose, cellulose acetate; powdered tragacanth; malt; gelatin; talc; stearic acid; magnesium stearate; calcium sulfate; vegetable oils, such as peanut oil, cottonseed oil, sesame oil, olive oil, corn oil and oil of theobroma; polyols such as propylene glycol, glycerol (glycerin), sorbitol, mannitol, and polyethylene glycol; agar, alginic acid; pyrogen-free water; isotonic saline; ethyl alcohol and phosphate buffer solutions, as well as other non-toxic compatible substances used in pharmaceutical formulations. Wetting agents and lubricants such as sodium lauryl sulfate, as well as coloring agents, flavoring agents and preservatives, can also be present

A pharmaceutical excipient or carrier is desirably employed in conjunction with the polyol fatty acid esters to provide desirable pharmaceutical compositions. Suitable carriers can provide a practical size to dosage relationship, composition forms which can be easily ingested and means for providing a very accurate unit dose in a convenient form. The pharmaceutical carrier can comprise from about 0.1% to 99% by weight of the total composition.

The following examples are illustrative of the invention.

### EXAMPLE 1

In-vitro study of the partition of unconjugated bilirubin between polyol fatty acid polyester and a micellar phase simulating duodenal milieu

This example relates to the determination of the binding capacity of the polyol fatty acid polyester used according to the invention, namely liquid sucrose polyester (SPE) towards bilirubin and the partition of bilirubin between an aqueous micellar phase simulating duodenal content and sucrose polyester in order to show the utility of sucrose polyester for the treatment of unconjugated hyperbilirubinemia.

The binding capacity of sucrose polyester for unconjugated bilirubin (Sigma, U.S.A.) was preliminary determined by adding unconjugated bilirubin dissolved in 0.5 ml of dimethyl sulfoxide (DMSO) or chloroform (Merck, Germany) to 10 ml of sucrose polyester (P&G, U.S.A.). After vigorous shaking followed by centrifugation for 10 minutes at 2000g the solution stayed bright yellow and transparent.

The transfer of unconjugated bilirubin from a micellar phase to the sucrose polyester phase was determined as follows: 5 ml of liquid sucrose polyester was placed in a screw-capped vial. The same volume of 87.8 µmol/l solution of unconjugated bilirubin in an aqueous phase consisting of 0.05 mol NaH₂PO₄ - 0.0125 mol Na₂HPO₄, pH 6.3, 10 mmol Na glycodeoxycholate, 3†mmol monoolein and 6 mmol oleic acid, with a Na⁺ concentration 0.15 mol, was added to the vial. The vial was sealed under nitrogen and shaken for 24 hours at 37°C. Simultaneously, a sample without sucrose polyester was used as a blank to find out the decomposition of bilirubin during 24 hr. of incubation. About 30 % of bilirubin was decomposed during a 24 hr. incubation period. Subsequently, a sample was centrifugated at 200,000g at 30°C for 30 minutes. Bilirubin was determined by a diazo method in the micellar phase and by direct spectrophotometry in chloroform solution in the sucrose polyester phase. Each experiment was 5 times repeated.

As shown above, unconjugated bilirubin formed a transparent solution or dispersion in sucrose polyester, when an excessive amount of sucrose polyester is mixed with a concentrated solution of bilirubin in DMSO as initial solvent.

### The following table summarizes the results obtained:

Partition of bilirubin (UCB) between the micellar phase and the polyol fatty acid polyester phase (SPE)

### [Fliessendes Objekt]

Thus, when sucrose polyester was exposed to a micellar solution of bilirubin, 96.2 ± 6.3 % (n=5) of bilirubin was recovered in the sucrose polyester phase. This was clearly evidenced by the yellow colour of the sucrose polyester phase versus the uncolored micellar phase.

The ability of the polyol fatty acid polyester to deprive the micellar phase of bilirubin is related to the distribution of bilirubin between this polyester and the micellar phase. The ratio of the concentration of bilirubin in said polyester phase and the micellar phase is an indicator of the ability of the polyol fatty acid polyester to extract bilirubin from the micellar phase and thus hinder its reabsorption and to stimulate intestinal excretion.

Thus, the above "in-vitro" experiment clearly shows that bilirubin almost quantitatively partitions into the polyol fatty acid polyester used according to the present invention if incubated with the micellar phase simulating duodenal content more than 95 % of bilirubin is trapped within this polyester. Thus, the polyol fatty acid polyester traps bilirubin in the gastro-intestinal tract and can be used to decrease systemic levels of bilirubin by interruption of the entero-hepatic cycle and stimulation of intestinal excretion of bilirubin.

### EXAMPLE 2

To demonstrate the in vivo effect obtained according to the use of the present invention, a 36 days parallel, placebo-controlled, double blind clinical study with 93 volunteers was conducted as a part of a safety control program in order to determine the effect of the polyol fatty acid polyester (Olestra) on intestinal microflora. The conversion of bilirubin to urobilinogen was determined in fecal samples and taken as one of the study parameters. It has been found that the consumption of 24 g of said polyol fatty acid polyester a day in a breakfast meal for one month led to a 30 % to 40% reduction of urobilinogen content in stools. This was accounted to a partition of bilirubin into said polyester, so that bilirubin was not any more available for microbial conversion.

This study has shown that 30 % to 40 % of daily bilirubin production is trapped by the polyol fatty acid polyester in the intestinal lumen when 24 g of this polyester is administered in breakfast and excreted out of the body in feces. This massive reduction of body pool of bilirubin results in a considerable reduction of systemic bilirubin. Since bilirubin partitions into the polyol fatty acid polyester in dependence on its concentration, one can expect an even more pronounced effect in patients with pathological concentration of bilirubin. In this respect it has to be remembered that the se-rum concentration in 3-day old neonates is about 20 times higher than in adults; in Crigler-Najjar patients it is about 20 to 50 times higher than in controls.

Therefore, the use of the non-absorbable, non-digestible polyol fatty acid polyester according to the present invention provides for a substantial reduction of systemic bilirubin and thus provides a simple, safe and effective therapy of hyperbilirubinemia specifically in neonates.

## Claims

1. The use of a non-absorbable, non-digestible lipid, for the manufacture of a pharmaceutical or dietary composition for the treatment of hyperbilirubinemia.

2. The use according to claim 1, wherein the non-absorbable, non-digestible lipid is selected from the group consisting of polyol fatty acid polyesters, esters of tricarballylic acids, diesters of dicarboxylic acids, triglycerides of alpha-branched chain carboxylic acids, ethers and ether esters containing the neopentyl moiety, fatty polyethers of polyglycerol, alkyl glycoside fatty acid polyesters, polyesters of two ether linked hydroxypolycarboxylic acids, esters of epoxide extended polyols, polydimethyl siloxanes, esterified linked alkoxylated glycerols, and mixtures thereof.

3. The use according to claim 2, wherein the non-absorbable, non-digestible polyol fatty acid polyester is a polyol fatty acid polyester having at least 4 fatty acid ester groups, wherein the polyol is selected from the group consisting of sugars and sugar alcohols containing from 4 to 8 hydroxyl groups and wherein each fatty acid group has from about 8 to about 22 carbon atoms.

4. The use according to any one of claims 3, **characterized in that** the polyol fatty acid polyester contains no more than 2 free hydroxyl groups.

5. The use according to claim 3 or 4, **characterized in that** the fatty acid ester groups contain from about 14 to about 18 carbon atoms.

6. The use according to claim 5, wherein the sucrose fatty acid polyester is a member selected from the group consisting of C₁₄, C₁₆, and C₁₈ hexa , hepta- and octa-esters, and mixtures thereof.

7. The use according to any one of claims 3 to 6, wherein the non-absorbable, non-digestible polyol fatty acid polyesters are selected from the group consisting of polyol fatty acid polyesters that comprise sugar polyesters, sugar alcohol polyesters, and mixtures thereof.

8. The use according to claim 7, wherein sugars and sugar alcohols ofthe non-absorbable, non-digestible sugar polyesters and sugar alcohol polyesters are selected from the group consisting of erythritol, xylitol, sorbitol, glucose, and sucrose.

9. The use according to claims 3 to 8, wherein the non-absorbable, non-digestible polyol fatty acid polyestes is selected from the group consisting of sucrose tetraoleate, sucrose pentaoleate, sucrose hexaoleate, sucrose heptaoleate, sucrose octaoleate, sucrose hepta-and octaesters of unsaturated soybean oil fatty acids, canola oil fatty acids, cottonseed oil fatty acids, corn oil fatty acids, peanut oil fatty acids, palm kernel oil fatty acids, or coconut oil fatty acids, glucose tetraoleate, the glucose tetraesters of coconut oil or unsaturated soybean oil fatty acids, the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, and mixtures thereof.

10. The use according to claim 9, wherein the sucrose fatty acid polyester is a member selected from the group consisting of the hexaoleate, heptaoleate and octaoleate of sucrose, and mixtures thereof.

11. The use according to anyone of claims 1 to 10, **characterized in that** the polyol fatty acid polyester comprises a mixture of a liquid polyol fatty acid polyester as defined in claims 3 to 10 and a solid polyol fatty acid polyester having a complete melting point above about 37°C. wherein: a) the polyol has at least 4 hydroxyl groups, b) the ester groups of the solid polyol fatty acid polyester comprise (i) C₁₂ or higher unsaturated fatty acid radicals or a mixture of said unsatu- rated radicals and C₂ to C₁₂ saturated fatty acid radicals, and (ii) C₂₀ or higher saturated fatty acid radicals, the molar ratio of (i):(ii) is from about 1:15 to about 2:1, and c) at least 4 of the hydroxyl groups of the polyol are esterified, said mixture being semi-solid at body temperature and having a complete melting point above about 37°C.

12. The use according to claim 11, **characterized in that** said mixture comprises 50 to 98 % by weight, preferably 90 to 95 % by weight, of said liquid polyol fatty acid polyester, and 50 to 2 % by weight, preferably 10 to 5 % by weight of said solid polyol fatty acid polyester.

13. The use according to any one of claims 1 to 9, **characterized in that** the pharmaceutical or dietary composition further comprises sufficient fat-soluble vitamins selected from the group consisting of vitamin A, vitamin D, vitamin E and vitamin K, or mixtures thereof, to prevent abnormally low levels of any of said fat-soluble vitamins in the individual patients ingesting said composition.

14. The use according to any one of claims 1 to 10, **characterized in that** the pharmaceutical or dietary composition further comprises sufficient anti-anal leakage agent to prevent leakage of said polyol fatty acid polyester through the anal sphincter.

15. The use according to claim 14, **characterized in that** the anti-anal leakage agent is a member selected from the group consisting of: edible C₁₂ and higher saturated fatty acids, and their edible salts; edible, digestible sources of C₁₂ and higher saturated fatty acids; edible, non-absorbable, non-digestible solid polyol fatty acid polyesters having at least 4 fatty acid ester groups, wherein the polyol is selected from the group consisting of sugars and sugar alcohols containing from 4 to 8 hydroxyl groups and wherein each fatty acid group has from about 8 to about 22 carbon atoms; and edible, non-digestible esters of alpha-branched chain C₁₀-C₁₈ fatty acids.

16. The use according to claim 15, **characterized in that** the anti-anal leakage agent is seleted from the group consisting of edible C₁₂-C₂₄ saturated fatty acids and edible salts of C₁₂-C₂₄ saturated fatty acids and substantially saturated triglyceride esters of C₁₂-C₂₄ fatty acids, or mixtures thereof, preferably edible, position-specific trigylceride selected from the group consisting of: the 1-stearoyl, 1-palmitoyl, 1-arachidoyl and 1-behenoyl 2,3-dioleins; the 2-oleoyl 1,3-distearins, 1,3-dipalmitims, 1,3-diarachidins and 1,3-dibehenins; 1-oleoyl distearin; 1-palmitoyl distearin; 1-arachidoyl distearin; and 1-behenoyl distearin; and mixtures thereof.

17. The use according to claim 14, **characterized in that** the anti-anal leakage agent is seleted from the group consisting of hydrogenated palm oil and natural or synthetic cocoa butter.

18. The use according to any one of claims 1 to 10, **characterized in that** the pharmaceutical or dietary composition has a complete melting above 10°C.

19. The use according to any one of claims 1 to 15, **characterized in that** the pharmaceutical composition is in the form of a medicament or a dietary composition.

20. The use according to claim 15, **characterized in that** the pharmaceutical or dietary composition is in the form of orally administrable drops, liquids or capsules, or a dietary preparation comprising said polyol fatty acid polyester or polyol fatty acid polyester mixture, and, if desired said vitamins, said anti-anal leakage agent, and usual pharmaceutically inert, non-toxic excipients and additives.

21. The use according to claim 15, **characterized in that** the dietary composition is in the form of milk, artificial mother milk and/or infant formula substitute.

22. The use according to any one of claims 1 to 15, **characterized in that** the pharmaceutical or dietary composition comprises dose units allowing the administration of said polyol fatty acid polyester or polyol fatty acid polyester mixture in a daily dose of 0.1 mg/kg to 500 mg/kg body weight.
